(19) Europäisches Patentamt

European Patent Office

Office européen des brevets

(11) **EP 1 058 847 B1**

(12) **EUROPEAN PATENT SPECIFICATION**

(45) Date of publication and mention
of the grant of the patent:
**31.07.2002 Bulletin 2002/31**

(21) Application number: **99963081.7**

(22) Date of filing: **14.12.1999**

(51) Int Cl.7: **G01N 33/00**
// G01N27:12

(86) International application number:
**PCT/US99/29621**

(87) International publication number:
**WO 00/37933 (29.06.2000 Gazette 2000/26)**

(54) **STOCHASTIC ARRAY PROCESSING OF SENSOR MEASUREMENTS TO DETECT AND QUANTIFY ANALYTES**

STOCHASTISCHE ARRAYVERARBEITUNG VON SENSORMESSUNGEN ZUR DETEKTION UND ZAHLENMÄSSIGER AUSWERTUNG VON ANALYTEN

TRAITEMENT MATRICIEL STOCHASTIQUE DE MESURES DE DETECTION POUR DETECTER ET QUANTIFIER DES ANALYTES

(84) Designated Contracting States:
**DE FR GB**

(30) Priority: **18.12.1998 US 216718**

(43) Date of publication of application:
**13.12.2000 Bulletin 2000/50**

(73) Proprietor: **Raytheon Company**
**El Segundo, California 90245-0902 (US)**

(72) Inventors:
• **GILBERT, Harold, C.**
**Placentia, CA 92670 (US)**
• **WURZBACH, James, A.**
**Altadena, CA 91001 (US)**

(74) Representative: **Steil, Christian, Dipl.-Ing. et al**
**Witte, Weller & Partner,**
**Rotebühlstrasse 121**
**70178 Stuttgart (DE)**

(56) References cited:
WO-A-93/03355          US-A- 5 417 100
US-A- 5 571 401

• **MASAYUKI NAKAMURA ET AL: "PATTERN RECOGNITION OF DYNAMIC CHEMICAL-SENSOR RESPONSES BY USING LVQ ALGORITHM" IEEE INTERNATIONAL CONFERENCE ON SYSTEMS, MAN AND CYBERNETICS. CYBERNETICS,US,NEW YORK, IEEE,1997, pages 3036-3041, XP000788314 ISBN: 0-7803-4054-X**
• **NAKAMURA M ET AL: "Application of plasma-polymer-film-coated sensors to gas identification using linear filters" SENSORS AND ACTUATORS B,CH,ELSEVIER SEQUOIA S.A., LAUSANNE, vol. 33, no. 1, 1 July 1996 (1996-07-01), pages 122-127, XP004013054 ISSN: 0925-4005**

**Description**

The present invention relates to an analyte monitoring system, comprising an array of sensor elements that produce a temporal response when exposed to a mixture of analytes from a target class, said sensor elements designed so that each of them responds to the same mixture with a different sensitivity so that said array can distinguish between different mixtures, and an array processor that embodies a stochastic signal processing algorithm in which each said sensor element is defined by a time-based model for the analytes in said target class, said time-bases model of the sensor element being based on Kalman filtering.

Further, the present invention relates to a method of quantifying concentration of analytes.

An analyte monitoring system as mentioned above is known from "Pattern Recognition of Dynamic Chemical-Sensor Responses by Using LVQ Algorithm" by M. Nakamura et.al. in IEEE International Conference on Systems, Man and Cybernetics; Cybernetics, US, New York, IEEE, 1997, pages 3036 - 3041.

This invention relates generally to array processing of sensor measurements and more specifically to a stochastic filtering technique for quantifying analytes in a target class in approximately real-time.

Air pollutant emissions are generated in such commercial settings as oil refineries, chemical plants, commercial paint spraying operations, and dry cleaner facilities. The oil refineries and chemical plants are characterized by large numbers of valves, flanges and fittings, each of which is a potential source of fugitive emissions. Typical facilities can have several hundred thousand of these devices, each of which is a potential source of a leak. Paint spraying and dry cleaning operations use chemicals in their processes, which pose a potential threat to employees and the surrounding environment.

The most common approach of monitoring vapor emissions is for a technician to patrol the area with a hand-held sensor such as a flame ionization detector to detect the presence of fugitive emissions in the atmosphere. If the total level of emissions is too high, air samples are drawn into bags for subsequent analysis, e.g., by gas chromatography, to determine the precise composition and relative concentrations of the different emission compounds in the atmosphere at that location. This process is labor intensive and thus expensive, localized to each individual test site, involves a significant delay between data gathering and analysis, is only performed occasionally, and is insensitive to either trace levels or gradual changes in vapor concentration.

As a result, low but potentially dangerous levels of emissions and gradual changes in vapor concentration that are indicative of some problem may go undetected for long periods of time during which both people and the environment are exposed. Furthermore, during this delay, the source of the emissions may deteriorate further. For example, in a large oil refinery valves may be checked as infrequently as once or twice a year. Thus, each leaky valve may be the source for considerable emissions in the atmosphere. Furthermore, if the valve continues to deteriorate if may present an explosion hazard. Similar hazards can occur in the paint spraying and dry cleaning operations if their air filtration systems should fail.

Recently a number of organic polymer based sensors have been developed for the purpose of sensing fugitive emissions, U.S. Patent No. 5,417,100 Reversible Sensor for Detecting Solvent Vapors to Miller, European Patent No. 0 596 973 B1 Device for Sensing Volatile Materials to Gardiner, and U.S. Patent No. 5,571,401 Sensor Arrays for Detecting Analytes in Fluids to Lewis. These sensors are cheaper than flame or photo ionization detectors, operate at lower power levels, and facilitate passive sensing. The patents suggest that these sensors may be used either individually or in arrays, in which the individual sensor elements have a predetermined intersensor variation such that different vapor compounds exhibit unique response signatures in a hand-held or localized emissions sensing system.

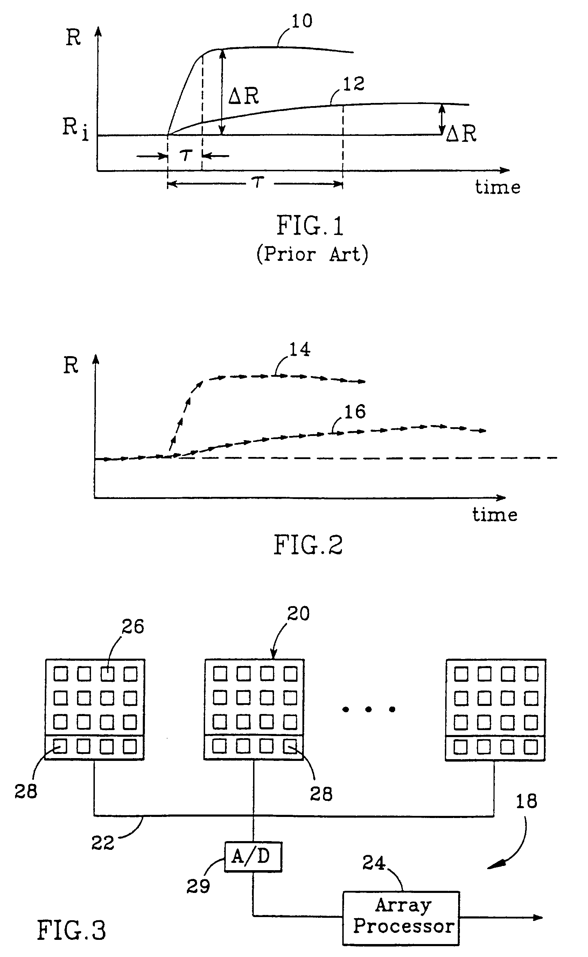

As shown in FIG. 1, the polymer based sensor exhibits an initial resistance $R_i$ in the ambient environment and responds to an event such as a change in the vapor composition and/or the relative concentration levels by changing its resistance by a unit step-response $\Delta R$. The magnitude of the step-response $\Delta R$ increases as the change in vapor concentration increases as shown by the response 10 to a large change in concentration as compared to the response 12 to a much smaller change in vapor concentration. For target vapor concentration levels on the order of thousands of ppm, currently available polymer sensors respond very quickly, exhibiting a time-constant $\tau$ of a few seconds for its resistance 10 to settle at the step-response level $R_i+\Delta R$. At lower concentration levels, however, the time-constant $\tau$ increases. As a result, the sensor resistance 12 will only reach the steady-state step response after hours or even days.

Known array processing systems are best used with very large arrays, in the hundreds or thousands of polymer based sensors, to detect and classify a large number of potential vapor mixtures at relatively high concentration levels. For example, "electronic noses" are under development for sniffing food or wine aromas to a) objectively maintain consistent produt quality or b) determine whether the food/wine has gone bad. The nose measures the unit step-response and normalizes it by the sensor's initial resistance $\Delta R/R_i$. At the high concentration levels typical in these applications (on the order of thousands of ppm), the sensor responds very quickly and reaches the steady-state step-response value in a matter of seconds. Thus, the required observation period for each sample can be limited to a few seconds.

The array of normalized step-responses is then processed to classify the aroma. The first array processing systems used a principal component analysis such as described by Lewis in U.S. Patent No. 5,571,401. Principal component analysis simplifies the presentation of the data and quantifies the distinguishing abilities of the individual sensors and the array as a whole. The linear combinations of the $\Delta R/R_i$ data are constructed such that the maximum variance is contained in the fewest mutually orthogonal dimensions. This greatly reduces the dimensionality of the data such that the resulting clustering of data provides a measure of the distinguishing ability of the sensor array. Currently, Lewis and other researchers are focusing their efforts on using Neural Networks. The neural networks are trained by exposing them to the step-response for known compositions/concentrations of vapors. The principal component and neural network systems have the advantage of being very simple, in the sense that they are based entirely upon the unit step-response of the sensor. This is very significant when dealing with array sizes in the thousands and target classes with hundreds of vapors.

The application of known array processing systems to the industrial setting would reveal certain deficiencies in the step-response techniques. First, the classification based systems do not quantify the relative concentrations of vapors in the mixture. Second, events that involve trace concentration levels or changes in concentration levels on the order of a few ppm, would require an observation period of hours or even days to measure the step-response. During the extended observation period, any changes in vapor concentrations in the interim would distort or completely mask the event. When dealing with the introduction of toxic vapors into an industrial setting where the real-time quantification of trace levels of these dangerous vapors is essential, the known array processing techniques are insufficient.

The above mentioned paper "Pattern Recognition ..." by M. Nakamura et.al. discloses a sensor system for providing qualitative information about a target gas. The sensor system comprises an array of sensors, each being a quartz-crystal microbalance sensor and coated with a different organic film. The sensors show various dynamic responses according to their respective target gases. Thus, the sensor array shows different response patterns according to the target gases. An LVQ algorithm is used to classify the responses of the array in real-time.

The signal processing of the dynamic sensor array responses is based on a linear model of each sensor, the coefficients of which being calculated on the basis of a Kalman filter algorithm.

In view of the above, it is the object of the invention to provide an improved analyte monitoring system and a method of quantifying concentrations of analytes.

This object is achieved by the analyte monitoring system, as mentioned at the outset, wherein said stochastic signal processing algorithm implements an extended Kalman filter that uses the sensors' time-based models, past measurements of the temporal responses, and a present measurement to compute an estimate of a state vector that includes the estimated quantities of the analytes in the target class, said Kalman filter using system and observation functions to update the state vector, said system function comprising the time-based model for each said sensor element and a time-based model for each said analyte in said target class, and said observation function defining how said state vector is transformed into the sensor response, so as to quantify the concentrations of the analytes in the target class present in the mixture.

The object is further achieved by a method of quantifying concentrations of analytes, comprising the steps of:

defining a target class of one ore more analytes;
providing an array of sensor elements that produce a temporal response when exposed to a mixture of analytes from the target class, said sensor elements designed so that each of them responds to the same mixture with a different sensitivity so that said array can distinguish between different mixtures, said sensor elements exhibiting time constants that increase as the concentration of said analytes is reduced;
modeling the response of each said sensor element as a non-linear time-based model for the analytes in said target class;
exposing the array to a mixture of analytes whose concentrations levels are so low that the time constants of the sensor elements' temporal responses are longer than an observation period in which to quantify the mixtures;
measuring the response of each said sensor element in the array; and
using the non-linear time-based models in conjunction with the measured responses to quantify the concentration of analytes in said target class present in said mixture, by extended Kalman filtering the sensors' time-based models, past measurements of the temporal responses, and the present measurement to compute an estimate of a state vector that includes the estimated quantities of the analytes in the target class, said extended Kalman filtering using system and observation functions to update the state vector, said system function comprising the time-based model for each said sensor element and a time-based model for each said analyte in said target class, and said observation function defining how said state vector is transformed into the sensor response.

In general, the present invention provides real-time quantification at low analyte concentrations.

This is accomplished by treating the array sensor measurements as a stochastic process, in which the sensor response to target analyte compounds is characterized by a time-based model. The sensors' temporal response, either continuous or sampled, is input to the array processor, which looks at the temporal behavior of the sensor array rather than its unit step-responses to quantify the vapor. In practical applications, temporal processing is critical at low concentrations due to the limitations imposed by the time constant of the sensor. The sensor response is just too slow to use conventional step-response systems. The array processor uses a processing technique, namely the state-based Kalman filter, to perform the classification and quantification. The Kalman filter provides the distinct advantage of providing real-time prediction whereas matched and Wiener filters have an associated delay.

These and other features and advantages of the invention will be apparent to those skilled in the art from the following detailed description of preferred embodiments, taken together with the accompanying drawings, in which:

FIG. 1, as described above, is a plot illustrating the unit step-response of a polymer based sensor;

FIG. 2 is a plot illustrating the temporal response of a polymer based sensor;

FIG. 3 is a block diagram of a stochastic array processor in accordance with the present invention;

FIG. 4 is a flowchart of an extended Kalman filter implementation of the stochastic array processor;

FIG. 5 is a plot of the actual vapor concentration and the values predicted by the Kalman filter;

FIGs. 6a and 6b illustrate the temporal responses of the pair of sensors that formed the array used by the Kalman filter in FIG 5; and

FIG. 7 is a diagram of a large vehicle painting system that is provided with a network of sensor arrays.

The present invention treats the problem of array processing of analyte sensor measurements as a stochastic process, in which the measurements are characterized by random variables in both time and state space. By comparison, the known step-response based systems treat the problem as a statistical instance or probabilistic event, in which the temporal nature of the sensor response is ignored. Temporal processing is particularly valuable when the sensor time constant exceeds the observation period, which is determined either by how long an event lasts or how long you can wait to measure the event. Under these conditions, known systems will either fail to provide the step-response or provide an inaccurate value.

By observing the temporal behavior of a sensor array, small responses in individual sensors that would otherwise be attributable to sensor noise or be indeterminate as to analyte quantity, can accurately quantify the analyte composition in a target class, e.g. a mixture of classes or species within a class, present in a mixture even at very low levels. With the proper processing, the temporal properties of the analyte can be tracked in approximately real-time. In general, analytes are defined as the compound of interest and include vapors that are detected by chemical sensors or pathogens such as bacteria or viruses that are detected by biological sensors. For purposes of illustration, the temporal processing algorithm will be described in conjunction with vapor sensor measurements.

FIG. 2 illustrates the response of a vapor sensor viewed from a temporal perspective. Rather than wait for the sensor to reach steady-state to measure its step-response, its resistance is sampled at a given rate and the raw data is passed to the array processor. When exposed to moderate to high concentration levels, the sensor response 14 changes quickly to reach its steady-state value. At these levels, the sensor's time constant is less than the observation period, and thus either step-response or temporal processing techniques would be adequate to classify the mixture. Notice, however, that several temporal samples can still be processed before the step-response could be measured.

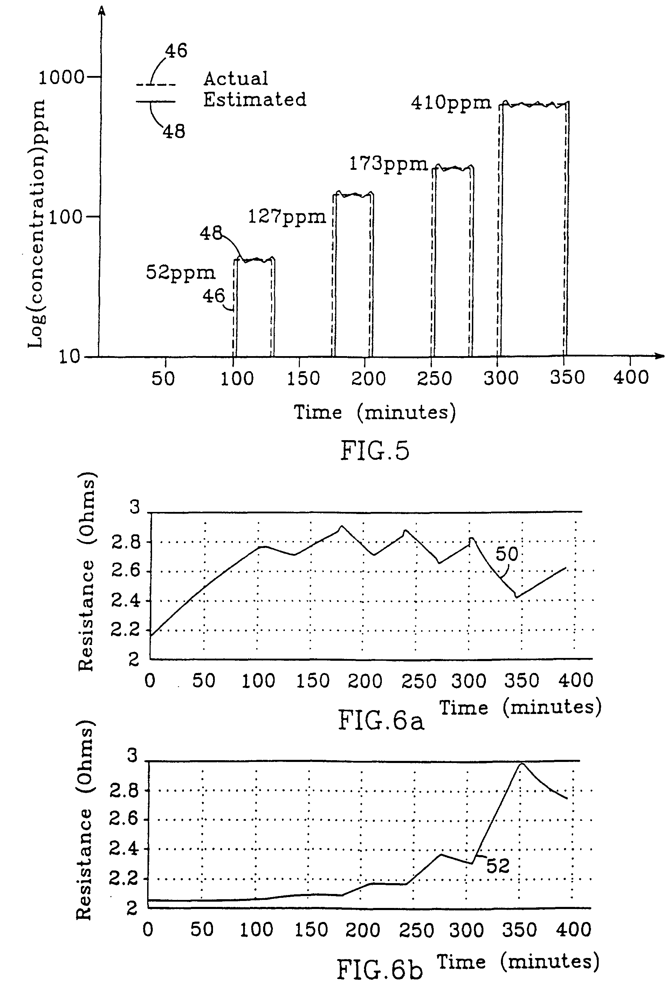

When exposed to low vapor levels, currently in the ppm range, the sensor response 16 changes very slowly, reaching its steady-state value, if ever, many hours or days later. In typical applications, the event would not last this long and almost certainly you would not want to wait hours or even days to detect and quantify the event. Observation periods of seconds or minutes are more practical. Using temporal based processing, the cause of the sensor response can be identified and tracked within the constraints of the observation period as shown in FIG. 5. Step-response systems would either fail or perform very poorly under this practical constraint.

As shown in FIG. 3, the monitoring system 18 includes a plurality of sensor arrays 20 that respond to the presence of a vapor mixture and transmit sampled raw data over a network 22 such as hard wired, RF, microwave or optical networks to an array processor 24. The sensor elements 26 are designed so that each of them responds to the same compound with a slightly different sensitivity. The inter-sensor deviations allow each array 20 to distinguish between different vapors in a target class and to detect their respective concentrations with reasonable accuracy. The arrays can be formed with a plurality of polymer sensors, metal oxide sensors, hybrids, or surface acoustic wave (SAW) sensors. Environmental sensors 28 sense the local ambient conditions including temperature, humidity, pressure and flow rate. This information can then be used to compensate the raw sensor data so that it accurately reflects the properties of the compounds, and not variations in the environmental conditions.

The sensor arrays 20 can be designed to detect the presence of volatile organic compounds (VOCs), inorganic vapors, fog, aerosols, smoke or water. The sensitivity of the sensor arrays and the sophistication of the signal conditioning can be designed to identify different classes of, for example, VOCs such as ketones, BTEX (Benzene-Toluene-Ethyl Benzene-Xylene), acetates, alcohols, and aliphatic hydrocarbons, or individual species within, for example, the BTEX class of VOCs such as Xylenes, Toluene and Ethyl Benzene. Thus, the target class can either represent multiple vapor classes or multiple species within a given class. Importantly, the number of vapors in the target class and array sizes are much smaller than those associated with the electronic nose, at present typical examples range from 6-12 vapors with array sizes from 12-48, respectively.

Array processor 24 is programmed with a stochastic algorithm such as the Kalman, matched, or Wiener filters, that takes the raw sensor data, suitably sampled and quantized by an A/D converter 29, processes it and quantifies the vapors in the target class present in the mixture. Embodied in the stochastic algorithm are time-based models for the sensor response whose form depends on the sensor and the specific processing algorithm. By comparing the measured temporal response to the time-based model, the array processor can accurately quantify low vapor concentrations on the order of 1 ppm in approximately real-time. Some small delay may be caused by system noise associated with the sensor and A/D quantizer, vapors outside the target class that look like noise, and the randomness of the target vapors themselves.

The first step is to define an analytic model for the sensor response function. One such model is defined by a first order linear differential equation in the saturation ratio (or site occupancy) of the sensor surface.

$$\dot{\Theta}(t) = \alpha\eta[1 - \Theta(t)] - \rho\Theta(t) \tag{1}$$

$\Theta$     surface saturation ratio $0 \leq \Theta \leq 1$

$\alpha$     adsorption rate coefficient (second$^{-1}$)

$\rho$     desorption rate (second$^{-1}$)

$\eta$     target vapor concentration (volume/volume) or partial pressure

The surface saturation ratio $\Theta$ represents the number of surface retention sites occupied by target vapor molecules as compared to the total number of sites that could be occupied. Thus it is a number that varies from zero (no sites occupied) to one (all sites full). The adsorption rate depends linearly on the target vapor concentration $\eta$, while the desorption rate $\rho$ is a constant. The solution of this differential equation reveals how the response depends on a time constant.

$$\Theta(t) = \frac{\alpha\eta}{\alpha\eta + \rho}\left[1 - e^{-(\alpha\eta + \rho)t}\right] + \Theta_0 e^{-(\alpha\eta + \rho)t} \tag{2}$$

$\Theta_0$     initial surface saturation ratio at t = 0

The reaction to a step function change in vapor concentration proceeds as $e^{-t/\tau}$ in which $\tau = 1/(\alpha\eta + \rho)$. The time constant $\tau$ is the time in which the response reaches $(e-1)/e$ of its final value. It takes 4.6 time constants to reach 99% of the final response. Obviously, as the vapor concentration $\eta$ deccreases so does the forward reaction rate $\alpha\eta$, and the time constant is increased. Thus, the sensor model responds much more slowly at lower vapor concentrations. The stochastic technique embedded in the invention is particularly important when the time-constant $\tau$ exceeds the observation period. $\Theta$, however, is not the sensor response but rather its saturation state. The response in terms of electrical conductivity depends on the saturation state according to a calibrating function $\phi$.

$$R(t) = \Omega(t) + \phi[\Theta(t)] \tag{3}$$

$\Omega$     sensor baseline resistance (ohm)

$\phi$     surface resistance calibrating function (ohm)

The polymer sensor has a finite conductivity even at zero saturation. This is represented by the baseline resistance $\Omega$. The total resistance R is the sum of the baseline resistance and the calibrating function of the saturation state. All of the mechanically conductive sensors presented in the literature and some of the chemically conductive sensors appear to conform to this model.

However, some types of chemically conductive sensors being developed exhibit a more complex behavior. These sensors appear to have two independent types of saturation states, one that responds very quickly but at a low level and one that responds much more slowly but at a higher level. Although the behavior has not yet been explained chemically, the sensors may undergo a change in surface conductivity that depends on the saturation state at the polymer surface, and simultaneously develop a volume conductivity that depends on the saturation state of the polymer mass. Regardless of the exact mechanism, the behavior can be modeled very accurately by introducing a second saturation state and differential equation.

$$\dot{\Theta}(t) = \delta\big[\Psi(t) - \Theta(t)\big] + \alpha\eta\big[1 - \Theta(t)\big] - \rho\Theta(t)$$
$$\dot{\Psi}(t) = \delta\big[\Theta(t) - \Psi(t)\big] \tag{4}$$

$\Psi$      volume saturation ratio $0 \le \Psi \le 1$
$\delta$      diffusion coefficient (second$^{-1}$)

Ordinarily, diffusion of the target vapor into the polymer mass would be governed by a parabolic partial differential equation which defines the diffusion coefficient. Here, we have approximated diffusion behavior with a uniform lumped mass. The calibration equation is modified for this binary state model as follows:

$$R(t) = \Omega(t) + \phi[\Theta(t), \Psi(t)] \tag{5}$$

The currently preferred approach for processing sensor array measurements is to use an extended Kalman filter. The Kalman filter uses model behavior and all past measurements together with the present measurement to compute an optimal estimate of the unknown system state quantities. The extended Kalman filter permits the method to be applied to nonlinear systems and provides conditionally optimal estimates.

The use of the extended Kalman filter in any particular application requires only the definition of the system function $\bar{f}(\bar{x}[k])$ and the observation function $\bar{h}(\bar{x}[k])$. In this case, the system function includes the time-based model for each sensor and a time-based model for each vapor in the target class. The observation function describes how the states are transformed into the sensor resistance.

First, the state vector of unknown system variables is defined as:

$$\bar{x} = \big|\eta_1\ \eta_2\ ...\ \Theta_{1,1}\ \Theta_{1,2}\ ...\ \Psi_{M,N}\big|^T \tag{6}$$

$\bar{x}$      system state vector.

The subscripted vapor concentrations $[\eta_1, \eta_2, ..., \eta_M]$ refer to water vapor, which is always present, and the target VOC vapors. The subscripted saturation states are identified for each sensor element [1,2,...,N] and target vapor [1,2,...,M]. Next, the nonlinear vector differential equation is given by:

$$\bar{x}(t) = \bar{f}[\bar{x}(t)] + \bar{w}(t) \tag{7}$$

$\bar{f}$      system function
$\bar{w}$      system noise.

System noise $\bar{w}$ drives the unknown values of the target vapor concentrations. Using a common Kalman filter technique, it is assumed that the noise sources are stationary and their statistics are known:

$$E\{\eta(t)\} = \hat{\eta}, \qquad E\{\eta(t)\eta(t + \tau)\} = \sigma_\eta^2 e^{-\omega_\eta|\tau|} \tag{8}$$

$\sigma_\eta^2$      excitation magnitude or variance of $\eta$
$\omega_\eta$      excitation rate (radian/second)

Under this assumption the vapor concentration may be modeled as the output of a lowpass filter driven by

Gaussian white noise.

$$\dot{\eta}(t) = -\omega_\eta \eta(t) + w_\eta(t) \tag{9}$$

$w_\eta$    white noise driving vapor concentration.

The differential relationships required to fully define the system function $\bar{f}$ are contained in equations 1, 4, and 9. The system noise $\bar{w}$ consists of random variables like $w_\eta$ whose statistics are known. To descretize the system equation 7, let $\bar{x}(t) \to \bar{x}[k]$ and replace the derivative with the first forward difference $\dot{\bar{x}}(t) \to (\bar{x}[k+1] - \bar{x}[k])/T$.

$$\bar{f}(\bar{x}[k]) = \begin{vmatrix} \eta_1[k](1 - \omega_{\eta 1}T) \\ \vdots \\ \alpha_{1,1}\eta_1[k]T + (1 - \delta_{1,1}T - \alpha_{1,1}\eta_1[k]T - p_{1,1}T)\Theta_{1,1}[k] + \delta_{1,1}T\Psi_{1,1}[k] \\ \vdots \\ \delta_{M,N}T\Theta_{M,N}[k] + (1 - \delta_{M,N}T)\Psi_{M,N}[h] \end{vmatrix}, \quad \bar{\omega}[k] = \begin{vmatrix} \omega_{\eta 1}[k] \\ \vdots \\ 0 \\ \vdots \\ 0 \end{vmatrix} \tag{10}$$

$T$    sample interval between steps $T = t_k - t_{k-1}$ (second)

The first part of the system function corresponds to the vapor concentration model in equation 9 for each vapor. The last part corresponds to the sensor model in equation 4 for each sensor.
Now define the observation vector $\bar{z}$ and its associated nonlinear observation function $\bar{h}$ at time $t_k$.

$$\bar{z}[k] = \begin{vmatrix} z_1[k] \\ z_2[k] \\ \vdots \\ z_N[k] \end{vmatrix}, \quad \bar{h}([k]) = \begin{vmatrix} \eta_1[k] \\ \Omega_1 + \phi_1(\Theta_{1,1}[k], \Psi_{1,1}[k], \cdots) \\ \Omega_N + \phi_N(\cdots, \Theta_{M,N}[k], \Psi_{M,N}[k]) \end{vmatrix} \tag{11}$$

$\bar{h}$    observation function
$\bar{z}$    observations.

The observation function relates how the states are transformed into resistance values in accordance with equation 3.
Here we have assumed that $z_1$ is the humidity measurement and that $h_1$ is the water vapor concentration. The observation vector consists of the ideal observations, derived from the ideal state vector, plus measurement error.

$$\bar{z}[k] = \bar{h}(\bar{x}[k]) + \bar{v}[k] \tag{12}$$

$\bar{v}$    measurement error contained in the observations.

Having defined the system and observation functions, the extended Kalman filter process is defined by five classical steps of computing 1) state prediction, 2) predicted error covariance matrix P, 3) Kalman gain matrix K, 4) corrected error covariance matrix P, and 5) corrected state vector as follows:

1. $\hat{x}[k|k-1] = \bar{f}\big(\hat{x}[k-1|k-1]\big)$

2. $P[k|k-1] = \big[\nabla\bar{f}\big(\hat{x}[k|k-1]\big)\big]P[k-1]\big[\nabla\bar{f}\big(\hat{x}[k|k-1]\big)\big]^{T} + Q$

3. $K[k] = P[k|k-1]\big[\nabla\bar{h}\big(\hat{x}[k|k-1]\big)\big]^{T}\Big(\big[\nabla\bar{h}\big(\hat{x}[k|k-1]\big)\big]P[k|k-1]\big[\nabla\bar{h}\big(\hat{x}[k|k-1]\big)\big]^{T} - R\Big)^{-1}$  (13)

4. $P[k|k] = P[k|k-1] - K[k]\big[\nabla\bar{h}\big(\hat{x}[k|k-1]\big)\big]P[k|k-1]$

5. $\hat{x}[k|k] = \hat{x}[k|k-1] + K[k]\big(\bar{z}[k] - \bar{h}\big(\hat{x}[k|k-1]\big)\big)$

$\hat{x}$     estimated system state
K     Kalman gain matrix
P     estimating error covariance
Q     process noise covariance
R     measurement error covariance

The conditional index k|k-1 indicates the one-step prediction at time $t_k$ based on the values at time $t_{k-1}$, while k|k indicates the corrected estimate at time $t_k$ based on the current observation $\bar{z}$. $\nabla\bar{f}(x[k-1|k-1])$ is the gradient of the system function with respect to the state vector evaluated at the prior state estimate. Similarly, $\nabla\bar{h}(x[k|k-1])$ is the gradient of the observation function with respect to the state vector evaluated at the currently predicted state. The estimating error covariance P is defined as the expected variation of the error between the estimated and actual states.

$$P[k] = E\Big\{\big(\hat{x}[k] - \bar{x}[k]\big)\big(\hat{x}[k] - \bar{x}[k]\big)^{T}\Big\}, \quad E\big\{\hat{x}[k] - \bar{x}[k]\big\} = 0 \qquad (14)$$

The process noise covariance Q is defined as the expected variation of the random forcing function as described in (7).

$$Q = E\{\bar{w}[k]\bar{w}^{T}[k]\}, \qquad E\{\bar{w}[k]\} = 0 \qquad\qquad (15)$$

If the inputs are assumed to be statistically independent, then Q is a diagonal matrix whose non-zero diagonal elements consist of the excitation magnitudes, $Q_{m,m} = E\{w_{\eta_m}^{2}\} = \sigma_{\eta_m}^{2}$. The measurement error covariance R is defined as the expected variation of the observations.

$$R = E\{\bar{v}[k]\bar{v}^{T}[k]\}, \qquad E\{\bar{v}[k]\} = 0 \qquad\qquad (16)$$

The processing steps of equation (13) are recursively repeated at each sampling interval, producing a current estimate of the sensor element states as well as the unknown vapor concentrations. In the prototype system, the sampling intervals are nominally five seconds apart. The system response, that is its ability to track changes in the vapor concentrations, depends on the numerous features of this process but is substantially faster than the previously employed total deflection processes.

It should be noted that the number and variety of analytic models of the sensor response is not limited by the processor. If new types of sensor elements are constructed that do not conform to the existing models (or differential equations), but their behavior can be accurately described by some other model, then it is a simple matter to append the new model to the existing processor. This will continue to occur in the future as new polymer compounds or new subject vapors are included in the array processing system.

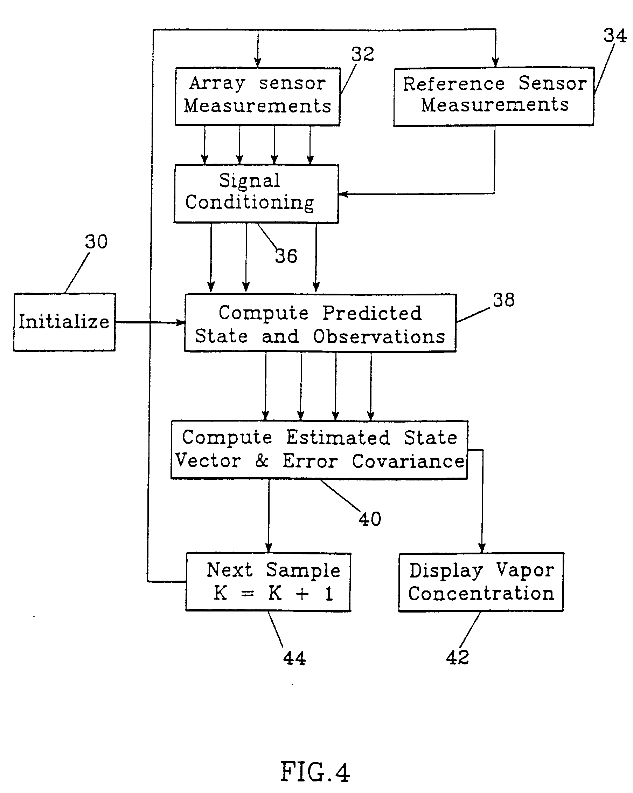

As illustrated in FIG. 4, the extended Kalman filter first initializes the sensor and vapor model parameters, the state vector, and the error covariance matrix (step 30). The sensor model parameters are typically found using any one of many well known non-linear optimization techniques, in which the parameters are adjusted to minimize some cost function such as mean-square error (mse). An array of N different sensors can typically be represented by a few different forms of differential equations with varying gain factors. The vapor model parameters include values for the excitation magnitude and rate. These parameters will depend greatly upon the specific application and may be updated over time using standard estimation techniques. One approach to initializing the state vector and error covariance matrix is described by H.W. Sorenson, "Kalman Filtering Techniques", Advances in Central Systems Theory and Applications, Vol. 3, 1996, pages 219-292.

Once initialized, the array and reference sensor measurements are triggered, steps 32 and 34 respectively. Thereafter, the raw sensor data is conditioned (step 36) to reject outliers, correct for reference measurements, etc. The conditioned data is then used to compute the predicted state and observations according to the sensor model and step one in eqn. 13 (step 38). Next, the corrected state vector is computed (step 40) in accordance with steps two through five of eqn. 13. The vapor composition and associated concentrations are read out of the corrected state vector and displayed (step 42). The Kalman filter proceeds to the next set of raw data samples and repeats indefinitely (step 44).

FIG. 5 is a plot of the actual temporal response 46 of a single target VOC and the estimated temporal response 48 generated by the extended Kalman filter using a two sensor array. In this experiment, 52 ppm of vapor was released at 100 min and turned off at 130 min, 127 ppm was released at 175 min and turned off at 205 min, 173 ppm was released at 235 min and turned off at 265 min, and 410 ppm was released at 295 min and turned off at 345 min. As shown, the stochastic processor accurately quantifies the target vapor at very low concentration levels, in approximately real-time and tracks the temporal response 46 very closely.

FIGs. 6a and 6b illustrate the responses 50 and 52 of the pair of sensors used to demonstrate the stochastic processing technique in FIG. 5. Due to the intersensor deviations, the sensors respond differently to the exposure to the same vapor. Although the response is much smaller and slower at the lower concentrations, the Kalman filter accurately and quickly tracks the temporal response of the vapor concentration as shown in FIG. 5. Also, note that the sensor responses are not even close to reaching steady-state when the next event occurs. In other words, the sensors' time-constant exceeds the effective observation period of the system. Thus, step-response systems would be ineffective.

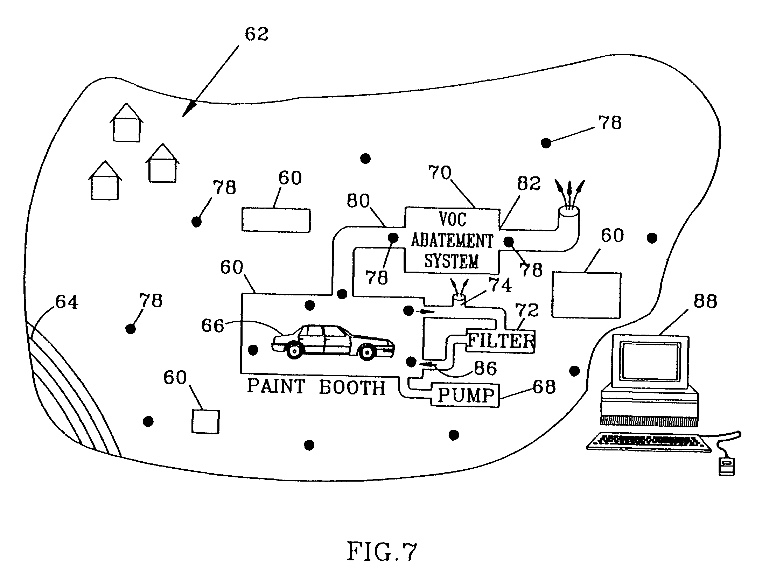

FIG. 7 illustrates the use of the present monitoring system in an industrial painting facility. A plurality of industrial paint booths 60 are distributed over an area, which happens in this example to lie upwind from a population center 62 and a river 64. The paint booths are used to manually paint entire vehicles 66, which produces effluents that are laden with VOCs from the evaporation of paint solvents and carriers. The booths must be ventilated to protect the personnel inside the booth. However, environmental concerns and regulations forbid the direct venting of effluent VOCs to the atmosphere.

As a result, each paint booth 60 is provided with a ventilation system that includes a pump 68 that draws clean air from the atmosphere into the booth and a VOC abatement system 70 that draws contaminated air from the booth, converts and/or destroys the VOCs using, for example, an oxidation process that burns the VOCs and then vents the cleansed air back to the atmosphere. Because the predominance of VOCs tend to settle in the bottom two-thirds of the booth, environmental regulations allow users to recirculate the air in the top one-third of the booth. This reduces the processing requirements, and thus cost, of the abatement system 70. As shown, the relatively clean air is recirculated through a filtration system 72 and then returned to the bottom of the booth. Even with the abatement and recirculation systems, the level of VOCs within the recirculation line may occasionally exceed an allowed safety threshold, in which case a bypass valve 74 opens to vent the contaminated air directly to the atmosphere rather than recirculating the VOC laden air back to the booth and exposing the personnel.

A VOC monitoring system 76 includes a plurality of sensor arrays 78 that are positioned a) at the inlet 80 and outlet 82 of the VOC abatement system 70, b) at the inlet 84 and outlet 86 of the recirculation system, c) inside the paint booth to measure ambient levels, d) at the bypass vent 74, and e) around the perimeter of the area. The sensors arrays are networked to a central monitoring unit 88 that is programmed with one of the stochastic array processing algorithms. The vapor concentration models and possibly the sensor models will vary depending upon the array location in the system. Because of their relatively close proximity, the sensor arrays on the booth and the abatement equipment can be hardwired, but the sensor arrays around the perimeter of the area are connected via a wireless network.

The sensor arrays on either side of the abatement system are used to compute the composition and concentration levels of the VOCs before and after abatement. This information is then used to control the operating parameters of the abatement system such as, in the oxidation process, the flow rate through the system, control the burn temperature, and control the burn period, which provides a safer and more efficient abatement process. The VOC composition (target class) can be resolved into different classes of VOCs such as ketones, BTEX, acetates, alcohols, and aliphatic hydrocarbons or can be resolved to the individual species such as xylenes, toluene, and ethyl benzene in the BTEX class. The sensor arrays on either side of the recirculation system are used similarly to monitor the quality of the circulated air and the efficiency of the filter.

The sensor arrays in the booth monitor the ambient VOC level. Should the total VOC concentration exceed the safety threshold, the central monitoring unit 88 will open the bypass vent 74 to quickly reduce the ambient VOC level. Since the bypass stream vents directly to the environment, it is essential to know what was released and in what quantity. The sensor unit at the bypass vent is used to compute the composition and concentration of the VOCs passed to the environment.

The bypass stream will form a plume that drifts in the wind toward the population center 62 and river 64. The sensor arrays 78 that are situated around the perimeter are used to track the progress of the plume as it moves toward the town and river. The sensor arrays measure the changing composition, and thus can tell whether certain components of the plume are sinking to the earth or are reacting with the atmosphere due to the presence of moisture, oxygen or sunlight. Furthermore, the sensor arrays measure the changing concentration, and thus can tell whether the plume is diluting to an acceptable level. This information plus current wind speed, and kinetic decomposition models for the target VOCs can be plugged into a dynamic flow model to predict what the VOC composition and concentration levels will be when the plume reaches the town and the river. This data can be critical in assessing any hazard to them. In addition, a gradient analysis can be performed on the flow data to identify which paint booth or booths are producing the VOC emissions.

While several illustrative embodiments of the invention have been shown and described, numerous variations and alternate embodiments will occur to those skilled in the art. Such variations and alternate embodiments are contemplated, and can be made without departing from the scope of the invention as defined in the appended claims.

**Claims**

1. An analyte monitoring system (18; 76), comprising:

   an array (20; 78) of sensor elements (26) that produce a temporal response (14, 16; 50, 52) when exposed to a mixture of analytes from a target class, said sensor elements (26) designed so that each of them responds to the same mixture with a different sensitivity so that said array (20; 78) can distinguish between different mixtures; and
   an array processor (24; 88) that embodies a stochastic signal processing algorithm (30-44) in which each said sensor element (26) is defined by a time-based model for the analytes in said target class, said time-based model of the sensor element (26) being based on Kalman filtering;

   **characterized in that** said stochastic signal processing algorithm (30-44) implements an extended Kalman filter that uses the sensors' time-based models, past measurements of the temporal responses, and a present measurement to compute an estimate of a state vector that includes the estimated quantities of the analytes in the target class, said Kalman filter using system and observation functions to update the state vector, said system function comprising the time-based model for each said sensor element (26) and a time-based model for each said analyte in said target class, and said observation function defining how said state vector is transformed into the sensor response, so as to quantify the concentrations of the analytes in the target class present in the mixture.

2. The system of claim 1, **characterized in that** said sensor elements (26) exhibit time constants ($\tau$) that increase as the concentration of said analytes is reduced and said array processor (24; 88) has an observation period in which to quantify the mixture, said stochastic signal processing algorithm (30-44) quantifying the concentrations when the concentrations are so low that the sensor elements time constants ($\tau$) are longer than the observation period.

3. The system of claim 2, **characterized in that** the stochastic signal processing algorithm (30-44) outputs a quantification (48) that tracks the temporal response of said mixture.

4. The system of claim 3, **characterized in that** the quantification (48) tracks the temporal response of said mixture in approximately real-time, limited only by the noise associated with the sensor array (20; 78), sensed analytes outside the target class, and the inherent randomness of the analytes in the target class.

5. A method of quantifying concentrations of analytes, comprising the steps of:

   defining a target class of one or more analytes;
   providing an array (20; 78) of sensor elements (26) that produce a temporal response (14, 16; 50, 52) when exposed to a mixture of analytes from the target class, said sensor elements (26) designed so that each of them responds to the same mixture with a different sensitivity so that said array (20; 78) can distinguish between different mixtures, said sensor elements (26) exhibiting time constants ($\tau$) that increase as the concentration of said analytes is reduced;

modeling the response of each said sensor element (26) as a non-linear time-based model for the analytes in said target class;

exposing the array (20; 78) to a mixture of analytes whose concentrations levels are so low that the time constants (τ) of the sensor elements' temporal responses are longer than an observation period in which to quantify the mixture;

measuring (32) the response of each said sensor element (26) in the array (20; 78); and

using (36-42) the non-linear time-based models in conjunction with the measured responses to quantify the concentration of analytes in said target class present in said mixture by extended Kalman filtering the sensors' timebased models, past measurements of the their temporal responses, and the present measurement to compute an estimate of a state vector that includes the estimated quantities of the analytes in the target class, said extended Ralman filtering using system and observation functions to update the state vector, said system function comprising the time-based model for each said sensor element (26) and a time-based model for each said analyte in said target class, and said observation function defining how said state vector is transformed into the sensor response.

**6.** The method of claim 5, comprising the step of reexposing the array (20; 78) to a different mixture of analytes prior to the sensor elements (26) settling to a steady-state value in response to the prior exposure.

**7.** The method of claim 5, wherein the quantification (48) tracks the temporal response of said mixture.

**8.** The method of claim 7, wherein the quantification (48) tracks the temporal response of said mixture in approximately real-time, limited only by the noise associated with the sensor array (20; 78), sensed analytes outside the target class, and the inherent randomness of the analytes in the target class.

**Patentansprüche**

**1.** Analyt-Überwachungssystem (18; 76), aufweisend:

einen Array (20; 78) aus Sensorelementen (26), die eine zeitliche Antwort (14, 16; 50, 52) erzeugen, wenn sie einer Mischung von Analyten aus einer Zielklasse ausgesetzt werden, wobei die Sensorelemente (26) so ausgelegt sind, daß jedes von ihnen auf dieselbe Mischung mit einer unterschiedlichen Empfindlichkeit antwortet, so daß der Array (20; 78) zwischen unterschiedlichen Mischungen unterscheiden kann; und

einen Arrayprozessor (24; 88), der einen Algorithmus (30-44) zur Verarbeitung von stochastischen Signalen aufweist, in dem jedes Sensorelement (26) durch ein zeitbasiertes Modell für die Analyte in der Zielklasse definiert ist, wobei das zeitbasierte Modell des Sensorelementes (26) auf Kalman-Filterung basiert;

**dadurch gekennzeichnet, daß** der Algorithmus (30-44) zur Verarbeitung von stochastischen Signalen ein erweitertes Kalman-Filter implementiert, das die zeitbasierten Modelle der Sensoren, vergangene Messungen der zeitlichen Antworten und eine vorliegende Messung verwendet, um eine Abschätzung eines Zustandsvektors zu berechnen, der die abgeschätzten Quantitäten der Analyte in der Zielklasse aufweist, wobei das Kalman-Filter eine System- und eine Beobachtungsfunktion verwendet, um den Zustandsvektor zu aktualisieren, wobei die Systemfunktion das zeitbasierte Modell für jedes Sensorelement (26) und ein zeitbasiertes Modell für jeden Analyt in der Zielklasse aufweist und wobei die Beobachtungsfunktion definiert, wie der Zustandsvektor in die Sensorantwort transformiert wird, um die Konzentrationen der Analyte in der Zielklasse zu quantifizieren, die in der Mischung vorhanden sind.

**2.** System nach Anspruch 1, **dadurch gekennzeichnet, daß** die Sensorelemente (26) Zeitkonstanten (τ) zeigen, die ansteigen, wenn die Konzentration der Analyte verringert wird, und daß der Arrayprozessor (24; 88) eine Beobachtungsperiode besitzt, in der die Mischung zu quantifizieren ist, wobei der Algorithmus (30-44) zur Verarbeitung von stochastischen Signalen die Konzentrationen quantifiziert, wenn die Konzentrationen so niedrig sind, daß die Zeitkonstanten (τ) der Sensorelemente länger sind als die Beobachtungsperiode.

**3.** System nach Anspruch 2, **dadurch gekennzeichnet, daß** der Algorithmus (30-44) zur Verarbeitung von stochastischen Signalen eine Quantifizierung (48) ausgibt, die die zeitliche Antwort der Mischung nachverfolgt.

**4.** System nach Anspruch 3, **dadurch gekennzeichnet, daß** die Quantifizierung (48) die zeitliche Antwort der Mischung annähernd in Echtzeit nachverfolgt, wobei dies lediglich begrenzt ist durch das Rauschen, das zugeordnet ist dem Sensorarray (20; 78), sensierten Analyten außerhalb der Zielklasse und der inhärenten Zufälligkeit der Analyte in der Zielklasse.

5.  Verfahren zum Quantifizieren von Konzentrationen von Analyten, mit den Schritten:

Definieren einer Zielklasse von einem oder mehreren Analyten;
Bereitstellen eines Arrays (20; 78) von Sensorelementen (26), die eine zeitliche Antwort (14, 16; 50, 52) erzeugen, wenn sie einer Mischung von Analyten aus der Zielklasse ausgesetzt werden, wobei die Sensorelemente (26) so ausgelegt sind, daß jedes von ihnen auf dieselbe Mischung mit einer unterschiedlichen Empfindlichkeit antwortet, so daß der Array (20; 78) zwischen unterschiedlichen Mischungen unterscheiden kann, wobei die Sensorelemente (26) Zeitkonstanten ($\tau$) zeigen, die ansteigen, wenn die Konzentration der Analyte verringert wird;
Modellieren der Antwort von jedem der Sensorelemente (26) als ein nicht lineares, zeitbasiertes Modell für die Analyte in der Zielklasse;
Aussetzen des Arrays (20; 78) einer Mischung von Analyten, deren Konzentrationspegel so niedrig sind, daß die Zeitkonstanten ($\tau$) der zeitlichen Antworten der Sensorelemente länger sind als eine Beobachtungsperiode, in der die Mischung zu quantifizieren ist;
Messen (32) der Antwort von jedem der Sensorelemente (26) in dem Array (20; 78); und
Verwenden (36-42) der nicht linearen, zeitbasierten Modelle in Verbindung mit den gemessenen Antworten, um die Konzentration von Analyten in der Zielklasse zu quantifizieren, die in der Mischung vorhanden sind, und zwar durch erweitertes Kalman-Filtern der zeitbasierten Modelle der Sensoren, der vergangenen Messungen ihrer zeitlichen Antworten und der vorliegenden Messung, um eine Abschätzung eines Zustandsvektors zu berechnen, der die abgeschätzten Quantitäten der Analyte in der Zielklasse beinhaltet, wobei der erweiterte Kalman-Filter-Vorgang eine System- und eine Beobachtungsfunktion verwendet, um den Zustandsvektor zu aktualisieren, wobei die Systemfunktion das zeitbasierte Modell für jedes der Sensorelemente (26) und ein zeitbasiertes Modell für jeden der Analyte in der Zielklasse aufweist und wobei die Beobachtungsfunktion definiert, wie der Zustandsvektor in die Sensorantwort transformiert wird.

6.  Verfahren nach Anspruch 5, mit dem Schritt, den Array (20; 78) einer unterschiedlichen Mischung von Analyten nochmals auszusetzen, and zwar bevor die Sensorelemente (26) in Antwort auf das vorherige Aussetzen auf einen gleichförmigen Zustandswert einschwingen.

7.  Verfahren nach Anspruch 5, wobei die Quantifizierung (48) die zeitliche Antwort der Mischung nachverfolgt.

8.  Verfahren nach Anspruch 7, wobei die Quantifizierung (48) die zeitliche Antwort der Mischung annähernd in Echtzeit nachverfolgt, begrenzt lediglich durch das Rauschen, das zugeordnet ist dem Sensorarray (20; 78), sensierten Analyten außerhalb der Zielklasse und der inhärenten Zufälligkeit der Analyte in der Zielklasse.

**Revendications**

1.  Système de surveillance d'analytes (18 ; 76), comprenant :

un groupement (20 ; 78) d'éléments de détection (26) qui produisent une réponse temporelle (14, 16 ; 50, 52) lorsqu'ils sont exposés à un mélange d'analytes provenant d'une classe cible, lesdits éléments de détection (26) étant conçus de telle sorte que chacun d'eux réagisse avec une sensibilité différente au même mélange, de telle manière que ledit groupement (20 ; 78) puisse établir des distinctions entre des mélanges différents ; et
un processeur-tableau (24 ; 88), qui met en oeuvre un algorithme de traitement stochastique de signaux (30 à 44), dans lequel chaque élément de détection (26) est défini par un modèle à base de temps pour les analytes de ladite classe cible, ledit modèle à base de temps de l'élément de détection (26) étant basé sur un filtrage de Kalman ;

**caractérisé en ce que** ledit algorithme de traitement stochastique de signaux (30 à 44) met en oeuvre un filtre de Kalman étendu, qui utilise les modèles à base de temps des détecteurs, les mesures passées des réponses temporelles et une mesure actuelle pour calculer une estimation d'un vecteur d'état, qui inclut les quantités estimées des analytes de la classe cible, ledit filtre de Kalman exploitant des fonctions du système et d'observation, pour la mise à jour du vecteur d'état, ladite fonction du système comprenant le modèle à base de temps pour chaque élément de détection (26) et un modèle à base de temps pour chacun desdits analytes de ladite classe cible, et ladite fonction d'observation définissant comment ledit vecteur d'état est transformé en une réponse du détecteur, de manière à quantifier les concentrations des analytes de la classe cible, présents dans le mélange.

2. Système selon la revendication 1, **caractérisé en ce que** lesdits éléments de détection (26) présentent des constantes de temps ($\tau$) qui croissent au fur et à mesure que la concentration desdits analytes est réduite, et **en ce que** ledit processeur-tableau (24 ; 88) a une période d'observation pendant laquelle le mélange doit être quantifié, ledit algorithme de traitement stochastique de signaux (30 à 44) quantifiant les concentrations lorsque les concentrations sont si basses que les constantes de temps ($\tau$) des éléments de détection sont plus longues que la période d'observation.

3. Système selon la revendication 2, **caractérisé en ce que** l'algorithme de traitement stochastique de signaux (30 à 44) produit une quantification (48) qui suit la réponse temporelle dudit mélange.

4. Système selon la revendication 3, **caractérisé en ce que** la quantification (48) suit la réponse temporelle dudit mélange approximativement en temps réel, les seules limites étant le bruit associé au groupement de détecteurs (20 ; 78), les analytes détectés qui n'appartiennent pas à la classe cible, et l'aspect aléatoire inhérent des analytes de la classe cible.

5. Procédé de quantification de concentrations d'analytes, comprenant les étapes de :

définition d'une classe cible d'un ou de plusieurs analyte(s) ;
mise en place d'un groupement (20 ; 78) d'éléments de détection (26) qui produisent une réponse temporelle (14, 16 ; 50, 52) lorsqu'ils sont exposés à un mélange d'analytes provenant de la classe cible, lesdits éléments de détection (26) étant conçus de telle sorte que chacun d'eux réagisse avec une sensibilité différente au même mélange, de telle manière que ledit groupement (20 ; 78) puisse établir des distinctions entre des mélanges différents, lesdits éléments de détection (26) présentant des constantes de temps ($\tau$) qui croissent au fur et à mesure que la concentration desdits analytes est réduite ;
modélisation de la réponse de chacun desdits éléments de détection (26) en tant que modèle à base de temps non linéaire pour les analytes de ladite classe cible ;
exposition du groupement (20 ; 78) à un mélange d'analytes dont les niveaux de concentration sont si bas que les constantes de temps ($\tau$) des réponses temporelles des éléments de détection sont plus longues qu'une période d'observation pendant laquelle le mélange doit être quantifié ;
mesure (32) de la réponse de chacun desdits éléments de détection (26) du groupement (20 ; 78) ; et
utilisation (36 à 42) des modèles à base de temps non linéaire en association avec les réponses mesurées, pour quantifier la concentration d'analytes de ladite classe cible, qui sont présents dans ledit mélange, au moyen d'un filtrage de Kalman étendu des modèles à base de temps des détecteurs, des mesures passées de leurs réponses temporelles et de la mesure actuelle pour calculer une estimation d'un vecteur d'état, qui inclut les quantités estimées des analytes de la classe cible, ledit filtrage de Kalman étendu exploitant des fonctions du système et d'observation, pour la mise à jour du vecteur d'état, ladite fonction du système comprenant le modèle à base de temps pour chaque élément de détection (26) et un modèle à base de temps pour chacun desdits analytes de ladite classe cible, et ladite fonction d'observation définissant comment ledit vecteur d'état est transformé en une réponse du détecteur.

6. Procédé selon la revendication 5, comprenant l'étape de réexposition du groupement (20 ; 78) à un mélange différent d'analytes, avant que les éléments de détection (26) ne se stabilisent à une valeur en régime permanent en réponse à l'exposition antérieure.

7. Procédé selon la revendication 5, dans lequel la quantification (48) suit la réponse temporelle dudit mélange.

8. Procédé selon la revendication 7, dans lequel la quantification (48) suit la réponse temporelle dudit mélange approximativement en temps réel, les seules limites étant le bruit associé au groupement de détecteurs (20 ; 78), les analytes détectés qui n'appartiennent pas à la classe cible, et l'aspect aléatoire inhérent des analytes de la classe cible.

FIG.1
(Prior Art)

FIG.2

FIG.3

FIG.4

FIG.5

FIG.6a

FIG.6b

FIG.7